# EUROPEAN PATENT SPECIFICATION

(11) **EP 1 472 985 B1**
(45) Date of publication and mention of the grant of the patent: **23.09.2009**
(21) Application number: 04009846.9
(22) Date of filing: 26.04.2004
(51) Int. Cl.: A61B 18/14

(54) **High frequency current treatment tool**
Behandlungsinstrument mit Hochfrequenzstrom
Outil de traitement de courant haute fréquence

(30) Priority: 28.04.2003 JP 2003123527
(43) Date of publication of application: 03.11.2004
(73) Proprietor: Olympus Corporation, Shibuya-ku, Tokyo (JP)
(72) Inventor: Suzuki, Keita, Kokubunji-shi, Tokyo (JP)
(74) Representative: von Hellfeld, Axel

(56) References cited:
- WO-A-03/005882
- US-B1- 6 273 887
- PATENT ABSTRACTS OF JAPAN vol. 018, no. 015 (C-1151), 12 January 1994 (1994-01-12) -& JP 05 253241 A (OLYMPUS OPTICAL CO LTD), 5 October 1993 (1993-10-05)

## Description

### BACKGROUND OF THE INVENTION

### Field of the Invention

The present invention relates to a high-frequency current treatment tool which performs treatment such as incision of a tissue by inserting it into a living organ and by supplying high-frequency current on the tissue.

### Description of Related Art

High-frequency current treatment tools are used for variety kinds of treatments together with an endoscope, and a high-frequency current forceps for excising a tissue by clamping the tissue using a tip portion of the forceps and by supplying high-frequency current on the tissue, is known as one of such high-frequency current treatment tools.

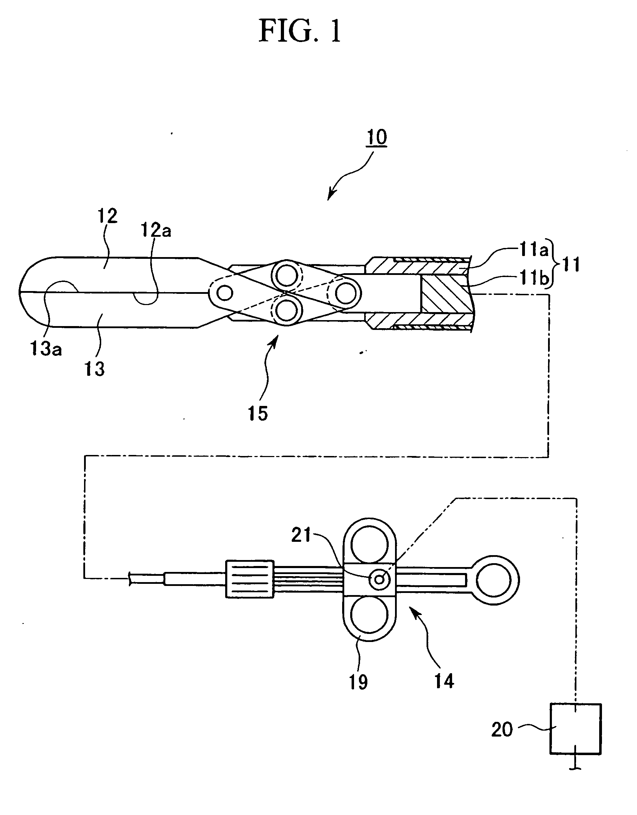

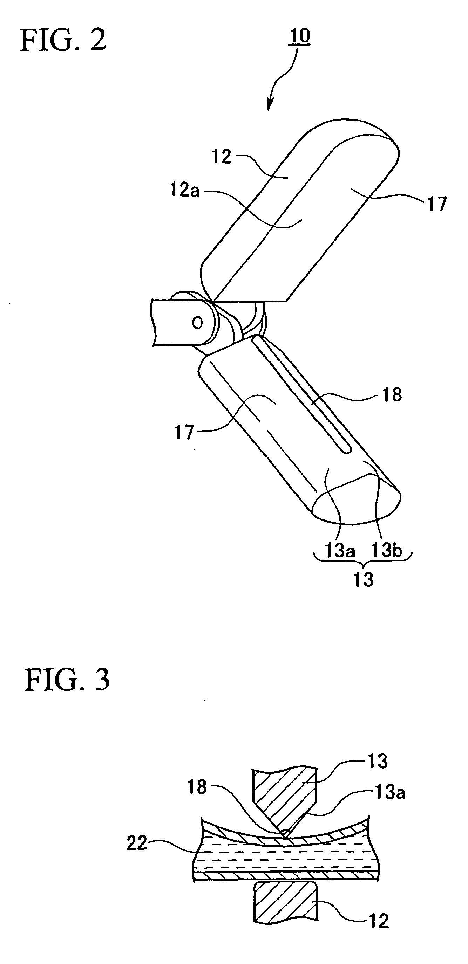

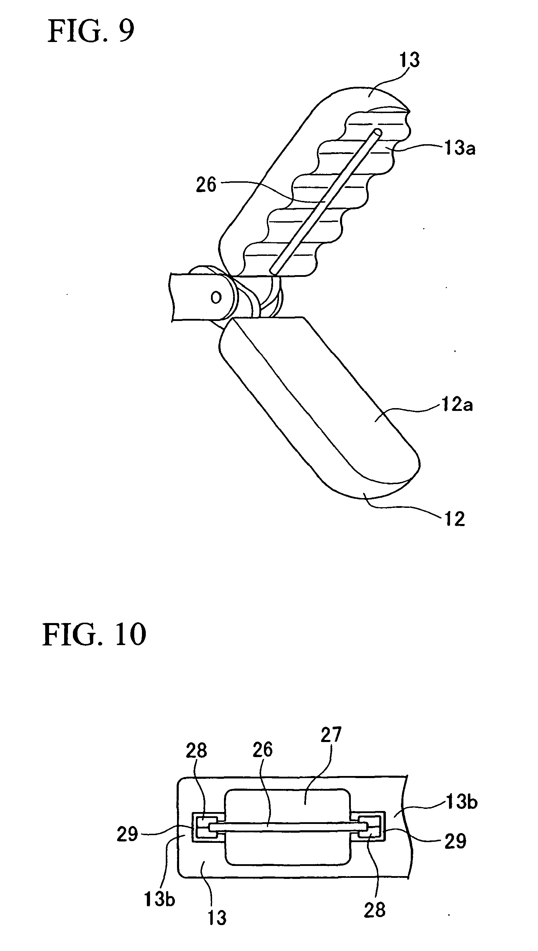

Conventionally, a forceps having electrodes on each clamp face formed on insulated clamp pieces (for example, refer to FIG. 2 of Japanese Unexamined Patent Application, First Publication No. Hei 5-253241), a forceps having insulated scissors-type clamp pieces and electrodes provided on each clamp face of the clamp pieces facing to each other (for example, refer to FIGS. 9 and 10 of U. S. Patent 5,827,281), etc., and a forceps having needle-shaped electrodes (for example, refer to FIG. 1 of Japanese Unexamined Patent Application, First Publication No. Hei 8-299355.), are proposed as the high-frequency current forceps.

Another example of a high-frequency treatment tool is described in granted United States Patent No. 6,273,887 B1.

### SUMMARY OF THE INVENTION

The present invention provides a high-frequency current treatment tool as defined in claim 1.

Thus, a high-frequency current forceps of the present invention includes: a pair of electrically insulated clamp faces facing each other; and a linear electrode provided on only one of the clamp faces.

According to this high-frequency current forceps, because the linear electrode is provided on only one of the clamp faces, the surface area of the electrode in the clamp face can easily be made smaller compare to a conventional one; therefore, current density can be increased. Furthermore, because a treatment part which contacts the electrode can be limited to one which contacts an internal area of the clamp face, safe operation can be performed by decreasing the likelihood of applying electrical damage to a living organ except for a location which contacts the electrode.

Therefore, according to this high-frequency current forceps of the present invention, because the electrode is provided on one of the clamp faces, current density can be more concentrated by making the surface area of the electrode smaller, and thus operation performance can be improved by easily and firmly operating on only a tissue which should be treated.

A corrugated portion is formed on only the clamp face not having the electrode.

The corrugated portion can prevent slipping when a tissue is clamped between the pair of clamp faces by increasing friction force. Therefore, the treatment can be done easily by firmly clamping the treatment part.

### BRIEF DESCRIPTION OF THE DRAWINGS

FIG. 1 shows not forming part of the invention a high-frequency current forceps.
FIG. 2 shows clamp pieces of the high-frequency current forceps not forming part of the invention.
FIG. 3 shows a treatment part clamped by the high-frequency current forceps not forming part of the invention.
FIG. 4 shows clamp pieces according to the present invention.
FIG. 5 shows another example of the clamp pieces according to the invention.
FIG. 6 is a cross sectional view of another example of the clamp pieces according to the invention.
FIG. 7 shows clamp pieces not forming part of the invention.
FIG. 8 shows a treatment part clamped by the high-frequency current forceps not forming part of the invention.
FIG. 9 shows clamp pieces according to another example not forming part of the invention.
FIG. 10 is a plan view of the high-frequency current forceps according to another example not forming part of the invention.

### DETAILED DESCRIPTION OF THE INVENTION

A high-frequency current forceps will be explained below referring to FIGS. 1 to 3.

As shown in FIG. 1, a high-frequency current forceps (a high-frequency current treatment tool) 10 has a flexible shaft member 11 inserted in a canal of an endoscope (not shown in the figures). A pair of clamp pieces 12 and 13 having a pair of clamp faces 12a and 13a facing to each other are provided at the tip side of the shaft member 11, and a controller 14 is provided at the bottom side of the shaft member 11.

The shaft member 11 has a flexible tube 11a and a control wire 11b inserted in the flexible tube 11a. A tip end of the control wire 11b is connected to the pair of clamp pieces 12 and 13 via a link mechanism 15. An outer periphery of the flexible tube 11a is covered with an electrical insulation cover.

The pair of clamp pieces 12 and 13 is made of metal such as stainless steel, and as shown in FIG. 2, the whole surface is electrically insulated by being covered with an insulation film 17.

The clamp face 12a is formed in planar shape, while the clamp face 13a has a ridge portion 13b extending along a length direction of the clamp piece 13, and forms a chevron shape.

An electrode 18 which is not covered with the insulation film 17 is provided on the ridge portion 13b of the clamp face 13a so that the electrode 18 forms a linear by exposing it.

One end of the electrode 18 is electrically connected to the flexible tube 11 a or the control wire 11b via a lead wire (not shown in the figures). The other end of the electrode 18 is provided within the clamp face 13a so that is does not protrude out from the outer shape of the clamp face 13a.

The controller 14 has a sliding controller 19 to which one end of the control wire 11b is connected, and a connection plug 21 for electrically connecting between the electrode 18 and one of the electrodes of a high-frequency wave power supply 20. Another electrode of the high-frequency wave power supply 20 (not shown in the figures) is connected to a skin of a human body so that a connection area between them is sufficiently larger than a connection area between a treatment part of the human body and the electrode 18.

Next, use of the high-frequency current forceps 10 having the above-mentioned constitution will be explained referring to FIG. 3.

Firstly, an endoscope (not shown in the figures) is inserted into a body cavity of a human body. Then, an injection needle (not shown in the figures) is inserted into the body cavity through the endoscope, and a treatment part 22 which should be excised is enlarged by injecting physiology salt solution into a lower layer of a mucous membrane of the treatment part 22. After that, the high-frequency current forceps 10 is inserted into the body cavity through the endoscope. At this time, the sliding controller 19 maintains its backward position, and the pair of clamp pieces 12 and 13 keeps their closed state.

Next, the high-frequency current forceps 10 is operated. By moving the sliding controller 19 toward the forward position, the link mechanism 15 is driven via the control wire 11b, and then the pair of clamp pieces 12 and 13 is opened. Then, after applying the clamp faces 12a and 13a on the enlarged treatment part 22, the sliding controller 19 is again pulled backward. Then, the link mechanism 15 is driven in an opposite direction, and the pair of clamp pieces 12 and 13 closes.

In this condition, when high-frequency current is supplied to the electrode 18 by controlling the high-frequency wave power supply 20, high-frequency current is supplied to another electrode (not shown in the figures) pasted to the human body, through the human body. At this time, current having very high electrical current density flows near around the electrode 18 because the electrode 18 is linear, and the surface area of the electrode 18 is sufficiently small. As a result, the living organ contacting the electrode 18 is excised. Moreover, because the surfaces of the clamp pieces 12 and 13 except for the place where the electrode 18 is installed, are insulated, current density in a tissue except for the place which contacts the electrode 18 becomes very small.

After the incision, the treatment part 22 is removed by removing the endoscope out from the body cavity with maintaining the treatment part 22 clamped.

According to the high-frequency current forceps 10, because the linear electrode 18 is only provided on the clamp face 13a, the surface area of the electrode 18 can easily be made smaller in relation to the conventional one, and therefore, performance of the incision can be improved by increasing the electrical current density.

Furthermore, because the electrode 18 is formed linearly and does not protrude from the clamp face 13a, and the exposed surface of the clamp piece 13 except for the location where the electrode 18 exits is insulated, a part of the treatment part 22 contacting the electrode 18 can be limited to one contacting an internal area of the clamp face 13a.

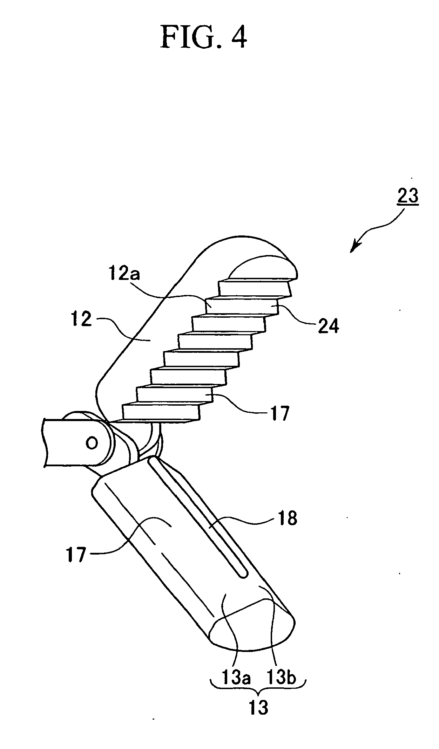

Next a high-frequency current forceps according to the present invention will be explained below referring to FIG. 4. Moreover, in the explanation below, as for the same components explained in the first embodiment, the same reference numbers will be used, and explanation thereof will be omitted.

The invention differs with the above examples in FIGS. 2-3 in the point that a corrugated portion 24 is formed on the clamp face 12a of the high-frequency current forceps 23, while the clamp face 12a of the high-frequency current forceps according to FIGS. 2-3 has a planar shape.

Other than the above, the high-frequency current forceps 23 has the same constitution as the high-frequency current forceps 10 according to FIGS. 2-3.

Next, use of the high-frequency current forceps 23 will be explained below.

In the same manner as for the high-frequency current forceps 10 according to FIGS. 2-3, an endoscope (not shown in the figures) having this high-frequency current forceps 23 is inserted into a body cavity. Next, the clamp pieces 12 and 13 clamp the treatment part 22 by controlling the sliding controller 19. At this time, even a slippery living organ can be firmly clamped without slipping because the corrugated portion 24 increases the surface area of the clamp face 12a. Under this condition, high-frequency current is applied on the electrode 18, and then the treatment part 22 is incised.

According to the high-frequency current forceps 23, the corrugated portion 24 prevents slipping when a tissue to be treated is clamped by the pair of clamp pieces 12 and 13. Therefore, an operation becomes easier because it is possible to clamp a living organ in a stable manner, and to firmly supply current on the desired treatment part 22.

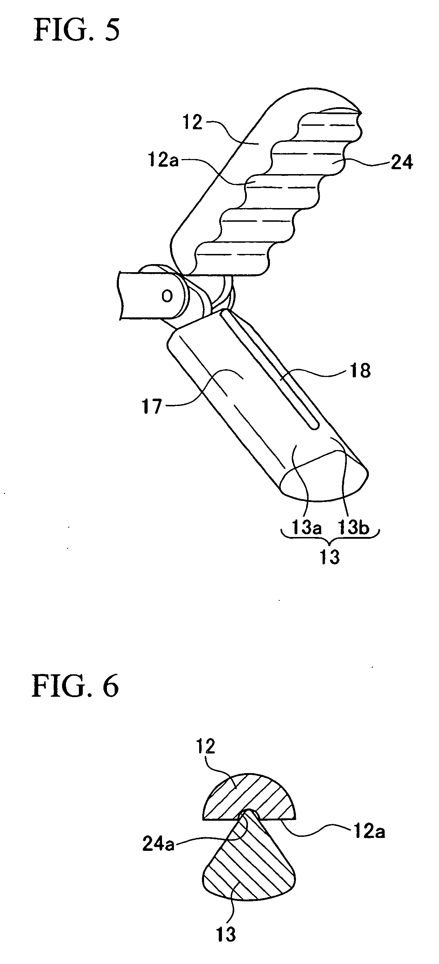

Moreover, the corrugated portion 24 can have a rounded shape as shown in FIG. 5.

By adopting such a rounded shape, it becomes possible to firmly clamp the treatment part 22, and to decrease a possibility of peeling off of the insulation film 17 formed on the surface of the clamp piece 12.

In addition, as shown in FIG. 6, a concave portion 24a which joins with the electrode 18 may be further provided along the center portion, in the width direction of the clamp face 12a, of the corrugated portion 24 formed on the clamp face 12a.

By providing the concave portion 24a, the electrode 18 can more strongly contact to the treatment part 22.

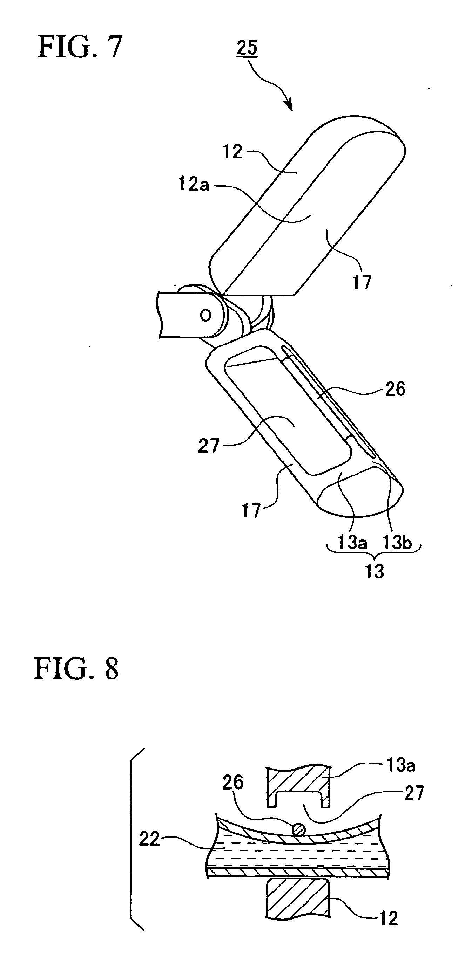

Next, a high-frequency current forceps will be explained below referring to FIGS. 7 and 8. Moreover, in the explanation below, as for the same components explained in the above-mentioned forceps, the same reference numbers will be used, and explanation thereof will be omitted.

The forceps differs with the invention in the point that a wire 26 is provided on the clamp face 13a at the high-frequency current forceps 25, while the linear electrode 18 is provided on the clamp face 13a at the high-frequency current forceps 23 according to the invention.

The high-frequency current forceps 25 has almost the same constitution as the high-frequency current forceps 10 according to FIGS. 2-3. However, a large concave portion 27 is formed on the clamp face 13a by removing a middle portion along the ridge 13b except for a tip side portion and a bottom side portion of the clamp piece 13.

Two ends of the wire 26 are supported by brazing them onto the clamp face 13a along the ridge 13b of the body 13a. Furthermore, the wire 18 is held over the concave portion 27.

A surface of the clamp face 13a and an inner face of the concave portion 27 of the clamp piece 13 are covered with the insulation film 17.

Next, use of the high-frequency current forceps 25 will be explained below.

In the same manner as in the high-frequency current forceps 10 according to the FIGS. 2-3, an endoscope (not shown in the figures) having this high-frequency current forceps 25 is inserted into a body cavity. Next, the clamp pieces 12 and 13 clamp the treatment part 22 by controlling the sliding controller 19. At this time, as shown in FIG. 8, a living organ of the treatment part 22 is clamped between the wire 26 and the clamp face 12a. At this time, physiology salt solution, etc., around the living organ diverges through the concave portion 27 without remaining around the wire 26.

In this condition, high-frequency current is supplied to the wire 26 by controlling the high-frequency wave power supply 25. At this time, incision of the treatment part 22 will be done in a short time because current density of current through the wire 26 becomes higher. In addition, because a concave portion 27 exists around the wire 26, physiology salt solution, etc., will not remain around the wire 26; therefore, lowering of the electrical current density due to divergence of current can be prevented.

According to the high-frequency current forceps 25, because the wire 26 is used as an electrode, the surface area of the wire 26 can easily be made smaller by adjusting its external diameter; therefore the electrical current density can be increased. In addition, the high-frequency current forceps 25 can perform incision in the most suitable condition for the tissue of the treatment part 22 by greatly concentrating current density.

Moreover, the shape of the clamp piece 13 is not limited to the one shown. For example, as shown in FIG. 9, it is possible to adopt the same shape as the clamp piece 12 which does not have the ridge 13b, and to provide a corrugated portion same as the second embodiment, and to provide the wire 26 above of the corrugated portion. In this case, the same action and the same effect can be obtained.

At this time, the wire 26 may be installed inside the corrugated portion, exposing only the surface. Otherwise, the wire 26 may be installed by exposing only a half thereof.

Moreover, the wire 26 is fixed by brazing the two ends thereof on the ridge 13b; however, the wire 26 may be fixed by an adhesive. Furthermore, as shown in FIG. 10, the wire 26 may be fixed by clamping the two ends with insulation members 28, and then inserting the two ends into supporting members 29 arranged on each end of the ridge 13b.

The invention is not to be considered as being limited by the foregoing description, and is only limited by the scope of the appended claim.

The clamp pieces 12 and 13 according to the invention are made of a metal such as stainless steel, etc., on which the surface is covered with the insulation film 17.

## Claims

1. A high-frequency current forceps (10; 23; 25) comprising:
a first clamp piece (13) having a first insulated clamp face (13a); a second clamp piece (12) having a second insulated clamp face (12a), the first and second clamp faces (13a, 12a) facing each other; and
a linear electrode (18) which is only provided on the first clamp face (13a), said first clamp face (13a) having a ridge portion (13b) formed along a longitudinal direction of the first clamp piece (13a), and forms a chevron shape, wherein the clamp pieces (12, 13) are made of metal,
the linear electrode (18) is arranged along the ridge portion (13b), **characterized in that**
a corrugated portion (24) is only formed on the second clamp face (12a), and
the whole surface of both clamp pieces (12, 13) is electrically Insulated by being covered with an insulation film (17) except for the electrode (18).

## Patentansprüche

1. Hochfrequenzstrom-Forceps (10; 23; 25) aufweisend:
ein erstes Klemmteil (13) mit einer ersten isolierten Klemmfläche (13a); ein zweites Klemmteil (12) mit einer zweiten isolierten Klemmfläche (12a), wobei die erst und die zweite Klemmfläche (13a, 12a) einander zugewandt sind, und
eine geradlinige Elektrode (18), die nur an der ersten Klemmfläche (13a) vorgesehen ist, wobei die erste Klemmfläche (13a) ein Gratteil (13b) aufweist, das entlang einer Längsrichtung des ersten Klemmteils (13a) gebildet ist und ein Winkelteil bildet, wobei die Klemmteile (12, 13) aus Metall bestehen, und die geradlinige Elektrode (18) entlang dem Gratteil (13b) angeordnet ist, **dadurch gekennzeichnet, dass**
ein geriffeltes Teil (24) nur auf der zweiten Klemmfläche (12a) gebildet ist, und die gesamte Oberfläche der beiden Klemmteile (12, 13), außer die Elektrode (18), durch einen Überzug mit einem Isolierfilm (17) elektrisch isoliert ist.

## Revendications

1. Pince à courant haute fréquence (10 ; 23 ; 25) comprenant :
une première pièce de clampage (13) ayant une première face de clampage isolée (13a) ; une deuxième pièce de clampage (12) ayant une deuxième face de clampage isolée (12a), la première et la deuxième faces de clampage (13a, 12a) se faisant face l'une l'autre ; et
une électrode linéaire (18) qui est disposée uniquement sur la première face de clampage (13a), ladite première face de clampage (13a) ayant une partie d'arête (13b) formée dans une direction longitudinale de la première face de clampage (13a), et forme une forme en chevron,
dans laquelle les pièces de clampage (12, 13) sont constituées de métal, l'électrode linéaire (18) est agencée le long de la partie d'arête (13b), **caractérisée en ce que** une partie striée (24) n'est formée que sur la deuxième face de clampage (12a), et la totalité de la surface des deux pièces de clampage (12, 13) est électriquement isolée en étant recouverte d'un film d'isolation (17) à l'exception de l'électrode (18).
